# EUROPÄISCHE PATENTSCHRIFT

(11) **EP 3 897 454 B1**
(45) Veröffentlichungstag und Bekanntmachung des Hinweises auf die Patenterteilung: **08.06.2022**
(21) Anmeldenummer: 19829539.6
(22) Anmeldetag: 19.12.2019
(51) Int. Cl.: A61F 2/07, A61F 2/966, A61F 2/24

(54) **VORRICHTUNG ZUM ZUFÜHREN UND SETZEN EINES IMPLANTATS IN EIN BLUTGEFÄSS**
DEVICE FOR FEEDING AND SETTING AN IMPLANT INTO A BLOOD VESSEL
DISPOSITIF POUR INTRODUIRE ET METTRE EN PLACE UN IMPLANT DANS UN VAISSEAU SANGUIN

(30) Priorität: 20.12.2018 WO PCT/EP2018/086246
(43) Veröffentlichungstag der Anmeldung: 27.10.2021
(73) Patentinhaber: Medizinische Universität Wien, 1090 Wien (AT)
(72) Erfinder: WISSER, Wilfried, 1090 Wien (AT); STELZMÜLLER, Marie-Elisabeth, 1090 Wien (AT)
(74) Vertreter: SONN Patentanwälte OG
(86) Internationale Anmeldenummer: PCT/EP2019/086153
(87) Internationale Veröffentlichungsnummer: WO 2020/127626

(56) Entgegenhaltungen:
- WO-A1-2016/164215
- US-A1- 2011 301 685
- US-B1- 6 514 280

## Beschreibung

Die Erfindung betrifft eine Vorrichtung zum Zuführen und Setzen eines zusammendrückbaren Implantats mit einem hohlzylindrischen Abschnitt aus einem expandierbaren Drahtmaschengeflecht mit einem ersten Ende und einem zweiten Ende in ein Blutgefäß, mit einer Hülse zur Aufnahme des Implantats in einem radial komprimierten Zustand, welche Hülse entlang einem Führungsdraht anordenbar ist, wobei die Hülse zur Aufnahme des Implantats im radial komprimierten Zustand durch eine innere Hülse gebildet ist, um welche innere Hülse zumindest eine äußere Hülse mit einem distalen Ende und einem proximalen Ende angeordnet ist.

Unter den Begriff Implantat fallen insbesondere Stents oder Gefäßstützen, Stentgrafts, Herzklappen oder dgl., welche in ein Blutgefäß eingebracht werden, um dieses offenzuhalten oder Funktionen innerhalb des Blutgefäßes oder Herzens zu unterstützen oder zu ersetzen. Den Implantaten gemeinsam ist ein hohlzylindrischer Abschnitt aus einem selbstexpandierenden Drahtmaschengeflecht, welches in radial komprimiertem Zustand in das Gefäß eingebracht wird und an der gewünschten Stelle im Gefäß in expandiertem Zustand verbleibt. Stents bestehen im Wesentlichen nur aus dem selbstexpandierenden Drahtmaschengeflecht und werden zum Offenhalten von Gefäßen in diese eingebracht. Stentgrafts kombinieren die Funktion eines Stents mit der Funktion einer Gefäßprothese, indem zumindest ein Teil des selbstexpandierenden Drahtmaschengeflechts mit einer Gewebeumhüllung versehen ist. Auch Herzklappen mit einem hohlzylindrischen Abschnitt aus einem selbstexpandierenden Drahtmaschengeflecht, innerhalb dessen die Herzklappe angeordnet ist, fallen unter den hier verwendeten Begriff der Implantate, auch wenn sie nicht im Blutgefäß selbst, sondern im Herzen angeordnet sind. Die Herzklappen stützen sich mit Hilfe des selbstexpandierenden Drahtmaschengeflechts an der gewünschten Stelle im Herzen ab und üben die entsprechende Klappenfunktion aus.

Neben Implantaten für Blutgefäße sind theoretisch auch andere zusammendrückbare Implantate für andere Gefäße oder Hohlorgane, wie zum Beispiel die Speiseröhre zur Stützung erkrankten Gewebes denkbar, welche mit der gegenständlichen Vorrichtung einsetzbar sind.

Beispielsweise stellt die interventionelle Versorgung von Pathologien von Blutgefäßen mithilfe von Stentgrafts ein etabliertes Verfahren dar. Zu den häufigsten Pathologien zählen Aneurysmen, also Erweiterungen des Querschnitts der Blutgefäße, und Dissektionen von Blutgefäßen, also Zerschichtungen der Gefäßinnenwand. Die akute Zerschichtung der aufsteigenden Körperschlagader, die sog. Typ A Dissektion der Aorta ascendens, ist ein akut lebensbedrohliches Zustandsbild, das einer sofortigen Operation bedarf. Die Operation erfordert ein Abkühlen des Patienten (Hypothermie) und eine Phase des Kreislaufstillstands, währenddessen das Gehirn selektiv mit Blut versorgt werden muss (antegrade Hirnperfusion). Die intraoperative Mortalität beträgt je nach Ausgangslage zwischen 15% und 30%. Grundsätzlich ist eine rasche Behandlung bzw. ein Abwenden der akuten Lebensbedrohung wünschenswert. Während die Versorgung der descendierenden Aorta mittels Stentgrafts häufig vorgenommen wird, sind für die Aorta ascendens kaum Implantate verfügbar und nur eingeschränkt und mit hohem Risiko verwendbar, da die exakte Positionierung der Gefäßprothese sehr nahe an der Aortenklappe und den Abgängen der Herzkranzgefäße (Koronarostien) problematisch ist.

Beispielsweise beschreibt die DE 10 2012 110 861 A1 eine endovaskuläre Stentprothese, welche auch für die Anordnung im Aortenbogen geeignet ist.

Die WO 2014/188412 A2 betrifft einen Stentgraft für die Aorta ascendens, bei der spezielle Verlängerungen mit Öffnungen für die Kopplung mit Herzkranzgefäßen vorgesehen sind. Die exakte Positionierung des Stentgrafts von den Leistengefäßen aus stellt eine extreme Herausforderung für den Chirurgen dar. Zusätzlich stellt die Überwindung der Krümmung des Aortenbogens, insbesondere bei zerschichteten Gefäßen ein hohes Risiko dar. Zusätzlich ist der Stentgraft extrem komplex aufgebaut und müsste für die Anatomie des jeweiligen Patienten speziell angefertigt werden, was einen Einsatz im Akutfall nicht zulässt.

Schließlich zeigt die US 2009/0264993 A1 eine Gefäßprothese für die Aorta ascendens für die Anwendung bei einer Dissektion der Aorta mit einer Vielzahl von Ankern mit daran angeordneten Haken am proximalen Ende. Die Gestaltung des proximalen Endes erhöht jedoch die Gefahr einer Beschädigung der Gefäßinnenwand oder sogar der Aortenklappe. Die darin offenbarte Zuführvorrichtung für den Stentgraft erlaubt eine exakte Positionierung nur bei Implantation über den Aortenbogen.

Aus der US 2011/0301685 A1 ist eine Vorrichtung zum Zuführen und Setzen eines Implantats in ein Blutgefäß der gegenständlichen Art bekannt geworden. Dabei wird beim Setzen des Implantats die äußere Hülse in proximale Richtung geschoben und das Implantat, welches auf einem Schaft gelagert ist, ebenso proximal aus der stationären inneren Hülse gezogen.

Eine andere Ausführungsform einer Vorrichtung mit koaxialen Hülsen zum Zuführen und Setzen eines Implantats in ein Blutgefäß der gegenständlichen Art wird in der US 6,514,280 B1 beschrieben. Beim Setzen des Implantats in das Blutgefäß wird die innere Hülse in proximaler Richtung in die äußere Hülse geschoben, wodurch sich das Implantat von der entferntesten Stelle beginnend expandiert.

Bei allen bisherigen zugelassenen Verfahren zum Zuführen und Setzen eines Implantats in ein Gefäß, insbesondere eines Stentgrafts in die Aorta ascendens ist eine exakte Positionierung des Implantats an der gewünschten Stelle des Implantats, beispielsweise eine Positionierung des Stentgrafts knapp oberhalb der Aortenklappe problematisch und wird durch bisherige Implantate nicht oder nur unzureichend unterstützt. Darüber hinaus erfolgt die Zuführung eines Stentgrafts für die Aorta ascendens von den Leistengefäßen aus, wodurch eine Überwindung des Aortenbogens notwendig ist.

Die Aufgabe der vorliegenden Erfindung besteht daher darin, eine Vorrichtung zum Zuführen und Setzen eines Implantats in ein Blutgefäß bereitzustellen, durch welche eine exakte und einfache Positionierung des Implantats an der gewünschten Stelle im Blutgefäß möglich ist. Die Vorrichtung zum Zuführen und Setzen des Implantats soll möglichst einfach aufgebaut sein, um eine einfache und kostengünstige Herstellung zu ermöglichen und eine breite Anwendung, insbesondere im Notfall zuzulassen. Nachteile bekannter Vorrichtungen sollen verhindert oder zumindest reduziert werden.

Gelöst wird die erfindungsgemäße Aufgabe durch eine oben genannte Vorrichtung zum Zuführen und Setzen eines Implantats in das Blutgefäß, wobei das Implantat derart in der inneren Hülse angeordnet ist, dass das zweite Ende des hohlzylindrischen Abschnitts auf der dem proximalen Ende der äußeren Hülse zugewandten Ende angeordnet ist, sodass beim Setzen des Implantats die das Implantat umgebende innere Hülse in distaler Richtung in die äußere Hülse bewegbar ist, sodass sich das Implantat vom zweiten Ende beginnend expandiert. Die erfindungsgemäße Vorrichtung ermöglicht eine Einbringung eines Implantats in ein Blutgefäß mit einer einfachen und raschen exakten Positionierbarkeit des Implantats im Blutgefäß. Die exaktere Positionierung des Implantats beim Setzen in das Blutgefäß ist deshalb möglich, da das Implantat beginnend vom proximalen Ende sukzessive geöffnet und expandiert wird. Es findet die Entfaltung des Implantats also in Richtung des Zuführens der Vorrichtung in das jeweilige Blutgefäß statt. Die Vorrichtung zum Zuführen und Setzen des Implantats ist relativ einfach aufgebaut und kann somit kostengünstig hergestellt werden. Die Vorrichtung kann sowohl in Richtung des Blutstroms, also antegrad, als auch gegen die Richtung des Blutstroms, also retrograd, eingebracht werden. Die Vorrichtung unterstützt die Versorgung des Patienten insbesondere im Akutfall. Insbesondere das Setzen eines Stentgrafts für die Aorta ascendens ist mit der erfindungsgemäßen Vorrichtung vom Herzmuskel aus möglich, indem der linke Ventrikel perforiert und die Vorrichtung von der Herzspitze durch die Aortenklappe in die aufsteigende Aorta eingeführt wird. Dadurch kann der Zugang von den Leistengefäßen aus verhindert werden, wodurch die Überwindung der Krümmung des Aortenbogens und eine gefährliche Beschädigung des ohnehin beeinträchtigten Gefäßes entfällt. Darüber hinaus ist eine exaktere Positionierung des Stentgrafts beim Setzen vom Herzmuskel her möglich, da der Stentgraft beginnend von einer Lage knapp oberhalb der Aortenklappe sukzessive geöffnet und expandiert wird. Das Absetzen des Stentgrafts ist daher unabhängig von der Beschaffenheit (Größe, torquierter Verlauf, etc.) der Leistengefäße und der Ausdehnung der Dissektion möglich. Die erfindungsgemäße Vorrichtung erlaubt das Zuführen und Setzen, d.h. Öffnen des Stentgrafts und damit das Sichern der Dissektionslamelle beginnend direkt oberhalb der Aortenklappe in Richtung des Blutflusses (von proximal nach distal). Dadurch ist der riskante Bereich der Aortenwurzel gesichert und die Gefahr, die aus der Aortenwurzel abgehenden Herzkranzgefäße (Koronarostien) zu verlegen, deutlich reduziert.

Das distale Ende der äußeren Hülse der Vorrichtung zum Zuführen und Setzen des Implantats ist vorzugsweise abgerundet oder zugespitzt ausgebildet. Durch eine derartige konstruktive Maßnahme wird das Setzen des Implantats in das jeweilige Blutgefäß hindurch erleichtert.

Wenn am distalen Ende der äußeren Hülse der Zuführvorrichtung Öffnungen angeordnet sind, kann eine ungehinderte Blutströmung während des operativen Eingriffs durch die Vorrichtung gewährleistet werden. Darüber hinaus kann durch derartige Öffnungen das Gewicht der Vorrichtung verringert werden.

Vorteilhafterweise ist eine zweite äußere Hülse vorgesehen bzw. die äußere Hülse zweigeteilt ausgebildet. Durch eine derartige Zweiteilung kann eine größere Krümmung der gesamten Vorrichtung aufgrund der Längenteilung erzielt und dadurch der chirurgische Eingriff erleichtert werden.

Wenn ein Kupplungselement am proximalen Ende der äußeren Hülse und ein dazu komplementär gestaltetes Kupplungselement am distalen Ende der zweiten äußeren Hülse zum Kuppeln der äußeren Hülse mit der zweiten äußeren Hülse vorgesehen ist, können die beiden Teile der äußeren Hülse der Vorrichtung einfach lösbar miteinander verbunden werden.

Die Kupplungselemente können beispielsweise durch radiale Abstufungen und/oder durch Magnete gebildet sein. Auf diese Weise kann die aus zwei Teilen bestehende äußere Hülse gemeinsam in miteinander gekuppeltem Zustand an die gewünschte Stelle im Blutgefäß eingebracht werden und dann im Zuge des Setzens des Implantats einfach geteilt werden.

Die vorliegende Erfindung wird anhand der beigefügten Zeichnungen näher erläutert. Darin zeigen:
- Fig. 1: eine Seitenansicht eines Stents als ein mögliches Implantat in radial komprimiertem Zustand;
- Fig. 2: eine Seitenansicht des Stents gemäß Fig. 1 in radial expandiertem Zustand;
- Fig. 3: eine Seitenansicht eines Stentgrafts für die Aorta ascendens als weiteres Implantat in radial komprimiertem Zustand;
- Fig. 4: eine Seitenansicht des Stentgrafts gemäß Fig. 3 in radial expandiertem Zustand;
- Fig. 5: eine Seitenansicht einer biologischen Herzklappe als weiteres Implantat in radial komprimiertem Zustand;
- Fig. 6: eine Seitenansicht auf die Herzklappe gemäß Fig. 5 in radial expandiertem Zustand;
- Fig. 7: eine erfindungsgemäße Vorrichtung zum Setzen eines Implantats in ein Blutgefäß in schematischer und teilweise geschnittener Darstellung;
- Fig. 8 bis: Fig. 12 verschiedene Zustände während des Setzens des Implantats in ein Blutgefäß mit der Vorrichtung gemäß Fig. 7 in schematischer Darstellung;
- Fig. 13A: eine schematische Darstellung des Setzens eines Stents als Implantat in ein Blutgefäß, wobei der Stent radial komprimiert ist;
- Fig. 13B: eine weitere schematische Darstellung während des Setzens eines Stents in ein Blutgefäß, wobei sich der Stent bereits teilweise in expandiertem Zustand befindet; und
- Fig. 14: einen in der Aorta ascendens angeordneten Stentgraft als Implantat.

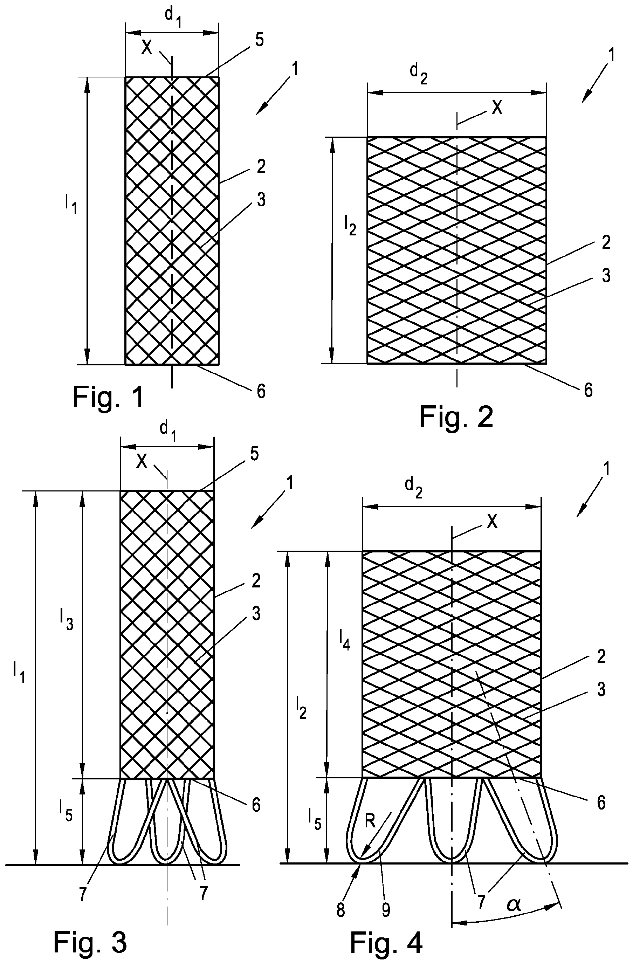

In Fig. 1 ist eine Seitenansicht eines Stents als Implantat 1 für ein Blutgefäß in radial komprimiertem Zustand dargestellt. Der Stent als Implantat 1 beinhaltet einen hohlzylindrischen Abschnitt 2 aus einem selbstexpandierenden Drahtmaschengeflecht 3, insbesondere aus einer Formgedächtnis-Legierung, vorzugsweise aus Nitinol. Der hohlzylindrische Abschnitt 2 weist ein erstes Ende 5 und ein zweites Ende 6 auf. Der Stent weist im radial komprimierten Zustand eine Gesamtlänge l₁ und einen Durchmesser d₁ auf.

Fig. 2 zeigt eine Seitenansicht auf den Stent gemäß Fig. 1 in radial expandiertem Zustand. Im expandierten Zustand weist der Stent eine Gesamtlänge l₂ und einen Durchmesser d₂ auf.

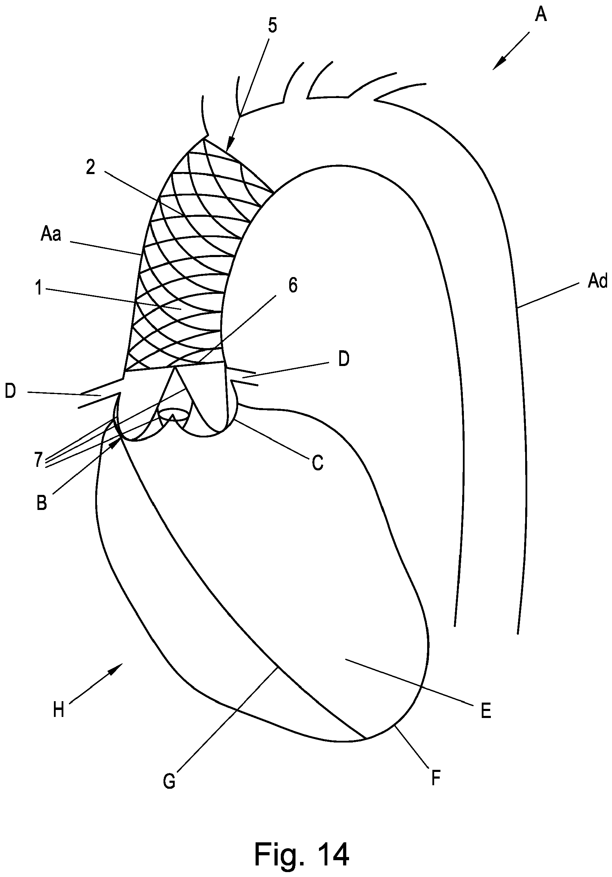

In Fig. 3 ist eine Seitenansicht eines Stentgrafts als weiteres mögliches Implantat 1 für die Aorta ascendens Aa in radial komprimiertem Zustand dargestellt. Der Stentgraft beinhaltet wie der Stent gemäß den Figuren 1 und 2 einen hohlzylindrischen Abschnitt 2 aus einem selbstexpandierenden Drahtmaschengeflecht 3, insbesondere aus einer Formgedächtnis-Legierung, vorzugsweise aus Nitinol. Der hohlzylindrische Abschnitt 2 ist mit einer Gewebeumhüllung 4, beispielsweise aus polymerem Fasergewebe, umgeben. Der hohlzylindrische Abschnitt 2 weist ein erstes Ende 5 und ein zweites Ende 6 auf. Am zweiten Ende 6 des hohlzylindrischen Abschnitts 2 des Stentgrafts, das ist jenes Ende 6, welches im implantierten Zustand zur Aortenklappe C (s. Fig. 14) weist, sind genau drei Distanzelemente 7 zum Einstellen des Abstands des zweiten Endes 6 des hohlzylindrischen Abschnitts 2 von der Aortenklappe C angeordnet. Die drei Distanzelemente 7 entsprechen der Anordnung der drei Taschen der Aortenklappe C, wodurch eine exakte Positionierung möglichst nahe an der Aortenklappe C bzw. der Kommissuren durch die vorgegebene Länge der Distanzelemente 7 garantiert wird. Der oberhalb der Distanzelemente 7 angeordnete hohlzylindrische Abschnitt 2 mit der Gewebeumhüllung 4 wird somit möglichst nahe oberhalb des Ansatzes der Aortenklappentaschen (Kommissurpfosten) in der Aorta ascendens Aa platziert. Ein solcher Stentgraft ist relativ einfach herstellbar und kann in unterschiedlichen Größen bereitgestellt werden, damit für den Akutfall das Implantat unmittelbar zur Verfügung steht.

Der Stentgraft weist im radial komprimierten Zustand vorzugsweise eine Gesamtlänge l₁ zwischen 50 mm und 100 mm und eine Länge l₃ des hohlzylindrischen Abschnitts 2 zwischen 40 mm und 90 mm auf. Der Durchmesser d₁ im radial komprimierten Zustand beträgt üblicherweise zwischen 5 mm und 12 mm. In Richtung der Längsachse X des Stentgrafts sehend weisen die Distanzelemente 7 eine Länge l₅ auf, welche entsprechend der Anatomie des Herzens zwischen 10 mm und 30 mm beträgt.

In dere Draufsicht sind die Distanzelemente 7 vorzugsweise in gleichmäßigen Winkelabständen von im Wesentlichen 120° angeordnet. Wie bereits oben erwähnt, sind bei anatomischen Fehlbildungen der Aortenklappen (bicuspide Aortenklappen) auch Varianten mit zwei Distanzelementen 7 mit Winkelabständen von 180° möglich (nicht dargestellt).

Fig. 4 zeigt eine Seitenansicht auf den Stentgraft gemäß Fig. 3 in radial expandiertem Zustand. Im expandierten Zustand weist der Stentgraft eine Gesamtlänge l₂ von vorzugsweise 40-90 mm und einen Durchmesser d₂ zwischen 20 mm und 60 mm auf. Der hohlzylindrische Abschnitt 2 weist eine Länge l₄ zwischen 30 mm und 80 mm auf. Im radial expandierten Zustand sind die Distanzelemente 7 vorzugsweise in einem Winkel α zwischen 10° und 30° abstehend angeordnet. Dadurch wird gewährleistet, dass die Taschen der Aortenklappe C beim gesetzten Stentgraft nicht in Ihrer Funktion beeinträchtigt werden. Das freie Ende 8 der Distanzelemente 7 ist vorzugsweise abgerundet ausgebildet, insbesondere mit einem Radius R zwischen 8 mm und 25 mm. Die Distanzelemente 7 sind vorzugsweise aus Drahtschlingen 9 gebildet, welche insbesondere aus dem selben Material wie das Drahtmaschengeflecht 3 des hohlzylindrischen Abschnitts 2 besteht. Dieses Material ist insbesondere eine Formgedächtnis-Legierung, vorzugsweise Nitinol, eine Nickel-Titan-Legierung. Dabei handelt es sich um spezielle Metalle, die vom komprimierten Zustand automatisch in eine expandierte Lage übergehen. Insbesondere Nitinol, eine Nickel-Titan-Legierung, stellt eine geeignete Formgedächtnis-Legierung dar, welche besonders elastisch, knickfest und flexibel ist.

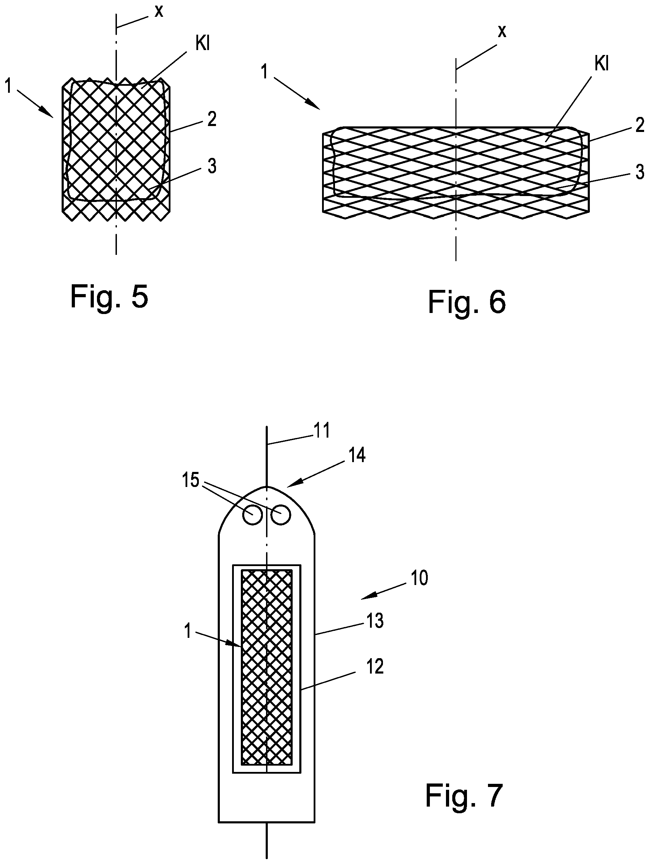

In Fig. 5 ist eine Seitenansicht einer biologischen Herzklappe als weiteres mögliches Implantat 1 in radial komprimiertem Zustand dargestellt. Die Herzklappe beinhaltet ebenso, wie ein Stent oder ein Stentgraft einen hohlzylindrischen Abschnitt 2 aus einem selbstexpandierenden Drahtmaschengeflecht 3, insbesondere aus einer Formgedächtnis-Legierung, vorzugsweise aus Nitinol. Am Drahtmaschengeflecht 3 befestigt befindet sich die Klappe Kl, insbesondere eine aus biologischem Material hergestellte Klappe.

Fig. 6 zeigt eine Seitenansicht der Herzklappe gemäß Fig 5 in radial expandiertem Zustand.

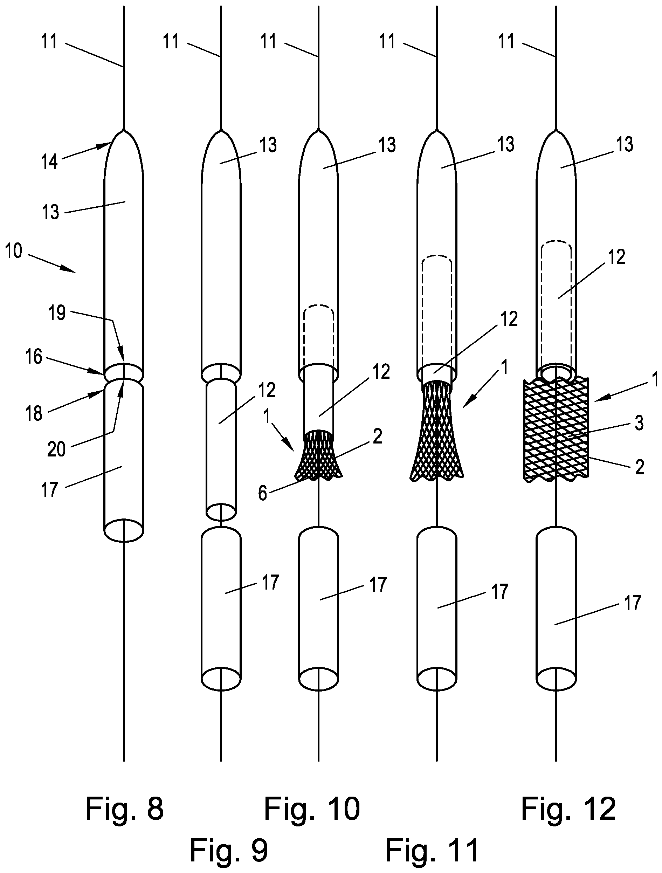

Fig. 7 zeigt eine erfindungsgemäße Vorrichtung 10 zum Setzen eines Implantats 1 in ein Gefäß oder einen Hohlkörper, insbesondere ein Blutgefäß. Zu diesen Implantaten 1 zählen Stents, wie in Fig. 1 und 2 dargestellt, Stentgrafts 1 gemäß Fig. 3 und 4 oder Herzklappen gemäß den Fig. 5 und 6. Die Vorrichtung 10 beinhaltet eine äußere Hülse 13 und eine innere Hülse 12 zur Aufnahme des Implantats 1 in radial komprimiertem Zustand. Die äußere Hülse 13 und die innere Hülse 12 werden entlang eines Führungsdrahts 11 angeordnet und an die gewünschte Stelle im jeweiligen Blutgefäß platziert. Das distale Ende 14 der äußeren Hülse 13 der Vorrichtung 10 zum Zuführen und Setzen des Implantats 1 ist vorzugsweise abgerundet oder zugespitzt ausgebildet. Durch eine derartige konstruktive Maßnahme wird das Setzen des Implantats 1 in das jeweilige Blutgefäß hindurch erleichtert. Wenn am distalen Ende 14 der äußeren Hülse 13 Öffnungen 15 angeordnet sind, kann eine ungehinderte Blutströmung während des operativen Eingriffs durch die Vorrichtung 10 gewährleistet werden. Darüber hinaus kann durch derartige Öffnungen 15 das Gewicht der Vorrichtung 10 verringert werden. Die Anzahl, Form und Größe der Öffnungen 15 kann variieren. Erfindungsgemäß dient die Vorrichtung 10 dazu, das Implantat 1 in das entsprechende Blutgefäß einzuführen und dort möglichst exakt zu platzieren und freizusetzen. Dabei wird beim Setzen des jeweiligen Implantats 1 die das Implantat 1 umgebende innere Hülle 12 in distaler Richtung in die äußere Hülse 13 bewegt, sodass sich das Implantat 1 vom zweiten Ende 6 des zylindrischen Abschnitts 2 beginnend expandiert. Dies wird nachfolgend anhand der Figuren 8-12 näher erläutert.

Die Fig. 8 bis Fig. 12 zeigen verschiedene Zustände während des Zuführens und Setzens des Implantats 1, beispielsweise eines Stents gemäß Fig. 1 und 2 in ein Blutgefäß in schematischer Darstellung. Bei diesem Ausführungsbeispiel der Vorrichtung 10 besteht die äußere Hülse aus zwei Teilen, nämlich der äußeren Hülse 13 und einer zweiten äußeren Hülse 17. Diese Ausführungsform verringert die Gesamtlänge der starren Hülse gegenüber einer einteiligen Ausführungsvariante. Wie in Fig. 8 dargestellt ist, wird die zweite äußere Hülse 17 mit dem proximalen Ende 16 der ersten äußeren Hülse 13 verbunden. Zur einfacheren Verbindung können entsprechende Kupplungselemente 19, 20 am proximalen Ende 16 der äußeren Hülse 13 bzw. am distalen Ende 18 der zweiten äußeren Hülse 17 angeordnet sein. Diese Kupplungselemente 19, 20 können durch radiale Abstufungen und bzw. oder Magnete (nicht dargestellt) gebildet sein. Das distale Ende 14 der äußeren Hülse 13 ist vorzugsweise abgerundet oder zugespitzt ausgebildet. Die in Fig. 8 dargestellte Vorrichtung 10 wird mit der darin enthaltenen inneren Hülse 12, die das Implantat 1 in radial komprimierten Zustand hält, in das Blutgefäß eingeführt.

Gemäß Fig. 9 wird nach dem Einführen der Vorrichtung 10 in das jeweilige Blutgefäß die zweite äußere Hülse 17 von der äußeren Hülse 13 entfernt, sodass die innere Hülse 12 mit dem darin befindlichen Implantat 1 in radial komprimierten Zustand freigelegt wird.

Danach wird entsprechend Fig. 10 die innere Hülse 12 in die äußere Hülse 13 geschoben und das Implantat 1 sukzessive vom zweiten Ende 6 des hohlzylindrischen Abschnitts 2 ausgehend sukzessive frei gesetzt. Dadurch kann das Implantat 1 exakt am gewünschten Ort, beispielsweise oberhalb einer Abzweigung eines Gefäßasts aus dem Blutgefäß, platziert werden.

Bei der Darstellung gemäß Fig. 11 ist das Implantat 1 fast vollständig freigelegt und die innere Hülse 12 der Vorrichtung 10 fast vollständig in der äußeren Hülse 13 angeordnet.

Entsprechend Fig. 12 wurde das Implantat 1 vollständig freigelegt, sodass dieser aufgrund des selbstexpandierenden Drahtmaschengeflechts 3 vollständig expandiert ist. Dadurch, dass nunmehr der Innendurchmesser des hohlzylindrischen Teils 2 des Implantats 1 gegenüber dem radial komprimierten Zustand entsprechend vergrößert ist, können nun die innere Hülse 12 und die äußere Hülse 13 nacheinander oder gleichzeitig durch das Implantat 1 und durch das Blutgefäß wieder entfernt werden.

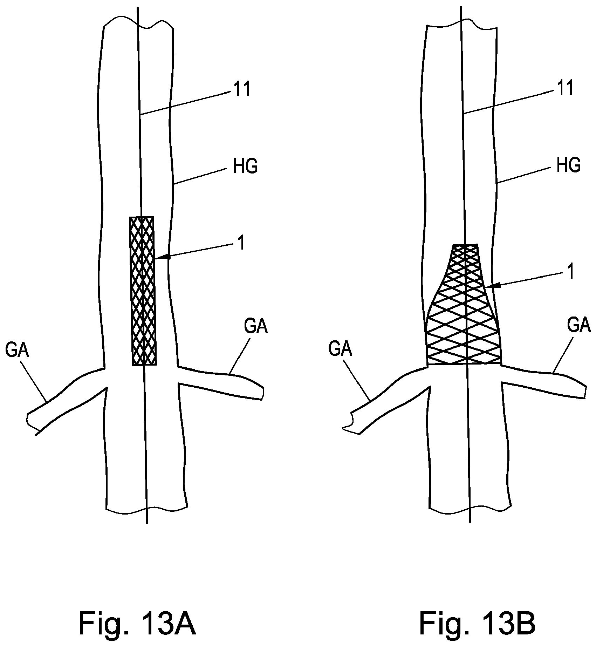

In Fig. 13 A ist das Setzen eines Stents als Implantat 1 in einem Hauptblutgefäß HG dargestellt. Das Hauptblutgefäß HG weist zwei Gefäßäste GA auf, oberhalb derer der Stent als Implantat 1 platziert werden soll. Die genaue Positionierung ist wichtig, da einerseits die Gefäßäste GA durch den Stent nicht verlegt werden soll, andererseits aber der Abstand des Endes des Stents von den Gefäßästen GA nicht zu groß sein soll, damit das geschädigte Hauptblutgefäß HG auch dort durch den Stent geschützt wird. Der Stent 1 wird mit der Vorrichtung 10 gemäß den Figuren 7 bis 12 entlang eines Führungsdrahtes 11 in das Hauptblutgefäß HG eingeführt.

Gemäß Fig. 13B wird exakt an der gewünschten Stelle des Hauptblutgefäßes HG die Vorrichtung 10 so bedient, dass eine Expansion des Stents vom proximalen Ende beginnend expandiert. Die Hülse der Vorrichtung 10 wird weiterbewegt, bis sich der Stent bis zum distalen Ende expandiert hat. Dadurch ist eine exakte Positionierung des Stents - hier genau oberhalb der Gefäßäste GA des Hauptgefäßes HG möglich. Schließlich wird die Vorrichtung 10 und der Führungsdraht 11 entfernt.

Schließlich ist in Fig. 14 ein in der Aorta ascendens Aa platziertes Stentgraft gemäß den Fig. 3 und 4 als Implantat 1 dargestellt. Die Abbildung zeigt den Herzmuskel H mit dem Septum G und der Aorta A, unterteilt in Aorta ascendens Aa und Aorta descendens Ad. Durch den anhand der Fig. 8-12 beschriebenen Absetzungsmechanismus kann der Stentgraft optimal in der Aorta ascendens Aa knapp oberhalb der Aortenklappe C und den Abgängen der Herzkranzgefäße D platziert werden. Die Länge des Stentgrafts wird so gewählt, dass der Bereich der Aorta ascendens Aa möglichst weit durch das Implantat abgedeckt wird. Durch die Distanzelemente 7 am zweiten Ende 6 des hohlzylindrischen Abschnitts 2 des Stentgrafts kann der Abstand des zweiten Endes 6 des hohlzylindrischen Abschnitts 2 mit der Gewebeumhüllung 4 von der Aortenklappe C exakt eingestellt werden. Die nicht von der Gewebeumhüllung 4 umgebenen Distanzelemente 7 ermöglichen auch das Freihalten der Abgänge der Herzkranzgefäße D aus der Aortenwurzel D.

## Patentansprüche

1. Vorrichtung (10) zum Zuführen und Setzen eines Implantats (1) mit einem hohlzylindrischen Abschnitt (2) aus einem expandierbaren Drahtmaschengeflecht (3) mit einem ersten Ende (5) und einem zweiten Ende (6) in ein Blutgefäß (B), mit einer Hülse zur Aufnahme des Implantats (1) in einem radial komprimierten Zustand, welche Hülse entlang einem Führungsdraht (11) anordenbar ist, wobei die Hülse zur Aufnahme des Implantats (1) im radial komprimierten Zustand durch eine innere Hülse (12) gebildet ist, um welche innere Hülse (12) zumindest eine äußere Hülse (13) mit einem distalen Ende (14) und einem proximalen Ende (16) angeordnet ist, **dadurch gekennzeichnet, dass** das Implantat (1) derart in der inneren Hülse (12) angeordnet ist, dass das zweite Ende (6) des hohlzylindrischen Abschnitts (2) auf der dem proximalen Ende (16) der äußeren Hülse (13) zugewandten Ende angeordnet ist, sodass beim Setzen des Implantats (1) die das Implantat (1) umgebende innere Hülse (12) in distaler Richtung in die äußere Hülse (13) bewegbar ist, sodass sich das Implantat (1) vom zweiten Ende (6) beginnend expandiert.

2. Vorrichtung (10) nach Anspruch 1, **dadurch gekennzeichnet, dass** das distale Ende (14) der äußeren Hülse (13) abgerundet ausgebildet ist.

3. Vorrichtung (10) nach Anspruch 1 oder 2, **dadurch gekennzeichnet, dass** am distalen Ende (14) der äußeren Hülse (13) Öffnungen (15) angeordnet sind.

4. Vorrichtung (10) nach einem der Ansprüche 1 bis 3, **dadurch gekennzeichnet, dass** eine zweite äußere Hülse (17) vorgesehen ist.

5. Vorrichtung (10) nach einem der Ansprüche 1 bis 4, **dadurch gekennzeichnet, dass** ein Kupplungselement (19) am proximalen Ende (16) der äußeren Hülse (13) und ein dazu komplementär gestaltetes Kupplungselement (20) am distalen Ende (18) der zweiten äußeren Hülse (17) zum Kuppeln der äußeren Hülse (13) mit der zweiten äußeren Hülse (17) vorgesehen ist.

6. Vorrichtung (10) nach Anspruch 5, **dadurch gekennzeichnet, dass** die Kupplungselemente (19, 20) durch radiale Abstufungen gebildet sind.

7. Vorrichtung (10) nach Anspruch 5, **dadurch gekennzeichnet, dass** die Kupplungselemente (19, 20) durch Magnete gebildet sind.

## Claims

1. Device (10) for feeding and setting an implant (1) that has a hollow-cylindrical portion (2) formed of an expandable wire mesh netting (3) with a first end (5) and a second end (6) into a blood vessel (B), with a sleeve for receiving the implant (1) in a radially compressed state, which sleeve is arrangeable along a guide wire (11), the sleeve for receiving the implant (1) in the radially compressed state being formed by an inner sleeve (12), around which inner sleeve (12) at least one outer sleeve (13) having a distal end (14) and a proximal end (16) is arranged, **characterised in that** the implant (1) is arranged in the inner sleeve (12) in such a way that the second end (6) of the hollow-cylindrical portion (2) is arranged at the end facing the proximal end (16) of the outer sleeve (13), such that, during setting of the implant (1), the inner sleeve (12) surrounding the implant (1) is movable in the distal direction into the outer sleeve (13), such that the implant (1) expands, beginning from the second end (6).

2. Device (10) according to claim 1, **characterised in that** the distal end (14) of the outer sleeve (13) is rounded.

3. Device (10) according to claim 1 or 2, **characterised in that** openings (15) are arranged at the distal end (14) of the outer sleeve (13).

4. Device (10) according to any of claims 1 to 3, **characterised in that** a second outer sleeve (17) is provided.

5. Device (10) according to any of claims 1 to 4, **characterised in that** a coupling element (19) is provided at the proximal end (16) of the outer sleeve (13), and a coupling element (20) shaped complementarily thereto is provided at the distal end (18) of the second outer sleeve (17) for coupling the outer sleeve (13) to the second outer sleeve (17).

6. Device (10) according to claim 5, **characterised in that** the coupling elements (19, 20) are formed by radial steps.

7. Device (10) according to claim 5, **characterised in that** the coupling elements (19, 20) are formed by magnets.

## Revendications

1. Dispositif (10) pour introduire et mettre en place un implant (1) avec une section cylindrique creuse (2) en un treillis métallique expansible (3) avec une première extrémité (5) et une deuxième extrémité (6) dans un vaisseau sanguin (B), avec un manchon pour recevoir l'implant (1) dans un état comprimé radialement, lequel manchon peut être disposé le long d'un fil de guidage (11), dans lequel le manchon pour recevoir l'implant (1) dans l'état comprimé radialement est formé par un manchon interne (12), manchon interne (12) autour duquel est disposé au moins un manchon externe (13) avec une extrémité distale (14) et une extrémité proximale (16), **caractérisé en ce que** l'implant (1) est disposé dans le manchon interne (12) de telle sorte que la deuxième extrémité (6) de la section cylindrique creuse (2) est disposée à l'extrémité tournée vers l'extrémité proximale (16) du manchon externe (13), de sorte que lors de la mise en place de l'implant (1), le manchon interne (12) entourant l'implant (1) peut être déplacé dans la direction distale dans le manchon externe (13), de sorte que l'implant (1) s'expanse à partir de la deuxième extrémité (6).

2. Dispositif (10) selon la revendication 1, **caractérisé en ce que** l'extrémité distale (14) du manchon externe (13) est arrondie.

3. Dispositif (10) selon la revendication 1 ou 2, **caractérisé en ce que** des ouvertures (15) sont pratiquées à l'extrémité distale (14) du manchon externe (13).

4. Dispositif (10) selon l'une des revendications 1 à 3, **caractérisé en ce qu'**un deuxième manchon externe (17) est prévu.

5. Dispositif (10) selon l'une des revendications 1 à 4, **caractérisé en ce qu'**un élément de couplage (19) est prévu à l'extrémité proximale (16) du manchon externe (13) et un élément de couplage de forme complémentaire (20) est prévu à l'extrémité distale (18) du deuxième manchon externe (17) pour le couplage du manchon externe (13) au deuxième manchon externe (17).

6. Dispositif (10) selon la revendication 5, **caractérisé en ce que** les éléments de couplage (19, 20) sont formés par des gradins radiaux.

7. Dispositif (10) selon la revendication 5, **caractérisé en ce que** les éléments de couplage (19, 20) sont formés par des aimants.
